(19) Europäisches Patentamt
European Patent Office
Office européen des brevets

(11) EP 0 827 736 B1

(12) **FASCICULE DE BREVET EUROPEEN**

(45) Date de publication et mention de la délivrance du brevet: **12.01.2000 Bulletin 2000/02**

(51) Int. Cl.7: **A61K 7/02**, A61K 7/00

(21) Numéro de dépôt: **97401816.0**

(22) Date de dépôt: **28.07.1997**

(54) **Utilisation d'une composition biphasique pour le démaquillage de compositions de maquillage sans transfert**

Verwendung einer zweiphasischen Zusammensetzung zum Abschminken von nichtübertragenden Schminkzusammensetzungen

Use of biphasic composition for the make-up removal of non-transfer make-up compositions

(84) Etats contractants désignés:
**DE ES FR GB IT**

(30) Priorité: **06.09.1996 FR 9610923**

(43) Date de publication de la demande:
**11.03.1998 Bulletin 1998/11**

(73) Titulaire: **L'OREAL**
**75008 Paris (FR)**

(72) Inventeur: **Picard, Elisabeth**
**78140 Velizy (FR)**

(74) Mandataire:
**Tezier Herman, Béatrice**
**L' OREAL,**
**D.P.I.,**
**6, rue Bertrand Sincholle**
**92585 Clichy Cédex (FR)**

(56) Documents cités:
**EP-A- 0 370 856**
**EP-A- 0 490 749**
**EP-A- 0 490 750**
**EP-A- 0 603 080**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 827 736 B1

## Description

L'invention a pour objet l'utilisation d'un démaquillant constitué de deux phases distinctes : une phase aqueuse et une phase huileuse, pour le démaquillage des compositions de maquillage sans transfert.

Les compositions de rouge à lèvres et de fond de teint comprennent généralement des corps gras tels que des cires et des huiles ainsi qu'une phase particulaire généralement composée de charges et de pigments.

Ces compositions, lorsqu'elles sont appliquées sur la peau ou les lèvres, présentent l'inconvénient de transférer, c'est-à-dire de se déposer au moins en partie, en laissant une trace, sur certains supports avec lesquels elles peuvent être mises en contact, et notamment un verre, une tasse, un vêtement ou la peau. Il s'ensuit une persistance médiocre du film sur la peau ou sur les lèvres nécessitant de renouveler régulièrement l'application de la composition de fond de teint ou de rouge à lèvres. Par ailleurs, l'apparition de traces inacceptables sur certains vêtements et notamment sur les cols de chemisier peut écarter certaines femmes à utiliser ce type de maquillage.

De façon récente, est apparue une nouvelle génération de produits de maquillage dits "non-transfert". Ces nouveaux produits se distinguent de ceux déjà connus par la présence d'huiles volatiles, à la place des huiles plus lourdes habituellement utilisées. En particulier, les compositions de rouge à lèvres et les fonds de teint non transfert comprennent des huiles de silicone qui n'étaient pas utilisées dans les compositions de rouge à lèvres de l'art antérieur.

La société Shiseido a envisagé dans sa demande de brevet JP-A-61-65809 des compositions de rouge à lèvres 《 sans transfert 》 contenant de 1 à 70% en poids de résine liquide de silicone à motifs répétitifs silicates (ou à réseau tridimensionnel) comportant des chaînes pendantes alkylées de 1 à 6 atomes de carbone ou phénylées, de 10 à 98% en poids d'une huile de silicone volatile à chaîne Si-O cyclique et à radicaux méthyle et des charges pulvérulentes.

Le brevet US-5,505,937 décrit des compositions 《 sans transfert 》 comprenant 1 à 70% d'une huile de silicone volatile, 0,1 à 15% d'une résine de silicone, 10 à 45% d'une cire, 5 à 50% de composés particulaires et 1 à 30% d'huile.

La demande de brevet français déposée sous le numéro 95-09254 concerne des compositions de maquillage 《 sans transfert》 comprenant l'association d'une huile siliconée phénylée et d'un composé volatil dans une phase grasse, et comprenant de préférence moins de 20% en poids d'huile hydrocarbonée non volatile.

La demande de brevet français déposée sous le numéro 96-07107 concerne des compositions de maquillage 《 sans transfert 》 comprenant une huile de silicone volatile et une cire de silicone solide ou semi-solide à température ambiante.

La demande de brevet français déposée sous le numéro 96-08420 concerne des compositions de maquillage 《 sans transfert 》 comprenant l'association d'un composé volatil et d'un composé gras pâteux afin de diminuer le transfert et/ou la migration, et/ou d'améliorer la tenue de ladite composition les comprenant.

Les huiles volatiles ayant tendance à s'évaporer rapidement, les nouvelles compositions de rouge à lèvres et de fond de teint ont la particularité de former un film de corps gras solide lorsqu'on les applique, respectivement sur la muqueuse labiale et sur la peau.

Contrairement aux compositions de rouge à lèvres de l'art antérieur qui ne nécessitent pas de démaquillage, les compositions de rouge à lèvres sans transfert doivent être ôtées en fin de journée par un démaquillage spécifique des lèvres. Or, on a constaté que les compositions de rouge à lèvres et de fond de teint sans transfert sont plus difficiles à démaquiller que des compositions de maquillage traditionnelles.

Il subsiste donc le besoin de disposer d'une composition permettant un démaquillage satisfaisant des compositions de maquillage sans transfert.

Or, la demanderesse a constaté avec étonnement que l'utilisation de compositions biphasiques pouvait permettre de démaquiller parfaitement et avec une grande facilité les maquillages sans transfert, dans des conditions de confort, en particulier de fraîcheur, très satisfaisantes.

Des compositions démaquillantes constituées de deux phases distinctes appelées aussi "compositions biphases" sont connues dans le domaine du démaquillage des yeux, par exemple par le document EP-A-370856. Toutefois, les produits de maquillage pour les yeux sont très différents des compositions pour le maquillage des lèvres et des fonds de teint.

En outre, les produits pour le maquillage des yeux s'appliquent par touches légères, en effleurant les cils ou la paupière avec le pinceau ou la brosse, tandis que les compositions de rouge à lèvres s'appliquent en exerçant une pression importante sur les lèvres, et les fonds de teint sont appliqués par massage de la peau, avec une certaine pression de la main. En outre, la muqueuse labiale présente une morphologie particulière : l'épiderme est fin et fragile et le derme est richement vascularisé et innervé. Ces caractéristiques morphologiques lui confèrent une affinité pour les produits de maquillage très différente de celles des paupières ou des fibres kératiniques constituant le cil.

En conséquence, rien ne laissait supposer que des compositions biphases utilisées pour démaquiller notamment les mascaras, étaient susceptibles d'être utilisées avec succès dans le démaquillage des compositions sans transfert.

L'invention a donc pour objet l'utilisation d'une composition constituée d'une phase aqueuse et d'une phase huileuse distinctes, pour le démaquillage des compositions de maquillage sans transfert.

L'invention s'applique de préférence à des compositions de maquillage qui peuvent se présenter sous la forme d'un produit de maquillage de la peau, en particulier un fond de teint, un fard à joues ou à paupières, ou un rouge à lèvres.

Elle s'applique en particulier aux compositions de maquillage décrites dans les documents JP-A-61-65809, US-5,505,937, FR-95-09254, FR-96-07107 et FR-96-08420.

L'invention s'applique plus particulièrement au démaquillage des compositions de rouge à lèvres et de fonds de teint sans transfert.
Elle s'applique plus spécialement aux compositions de maquillage comprenant une huile volatile, des corps gras et une phase particulaire.
En particulier, elle s'applique aux compositions comprenant :

- 1 à 90%, préférentiellement de 10 à 70% en poids d'une huile volatile, encore plus préférentiellement de 20 à 60% en poids d'une huile volatile,
- un composé éventuellement siliconé choisi parmi les cires de silicone, les résines de silicone, les huiles de silicone, éventuellement phénylées, un corps gras pâteux,
- jusqu'à 50% en poids d'une phase grasse constituée de corps gras usuels (cires et/ou huiles),
- de 0 à 30%, préférentiellement de 5 à 25% en poids d'une phase particulaire.

Les compositions de maquillage sans transfert auxquelles s'applique l'invention comprennent donc au moins une huile volatile, qui peut être choisie en particulier parmi les huiles hydrocarbonées ou les huiles siliconées, cycliques ou linéaires, seules ou en mélange.
Par huile volatile, on entend dans la présente description, toute huile susceptible de s'évaporer au contact de la peau.
De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

Parmi les huiles siliconées volatiles, on peut citer le cyclotétradiméthylsiloxane, le cyclopentadiméthylsiloxane, le cyclohexadiméthylsiloxane et le méthylhexyl-diméthylsiloxane. Parmi les huiles hydrocarbonées volatiles, on peut citer les isoparaffines.

La phase particulaire peut comprendre des pigments et/ou des nacres et/ou des charges habituellement utilisés dans les compositions cosmétiques.
Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.
Les pigments peuvent être présents dans la composition à raison de 0 à 15% en poids de la composition finale, et de préférence à raison de 8 à 10% en poids. Ils peuvent être blancs ou colorés, minéraux et/ou organiques, de taille usuelle ou nanométrique. On peut citer les dioxydes de titane, de zirconium ou de cérium, ainsi que les oxydes de zinc, de fer ou de chrome, le bleu ferrique, le noir de carbone, les outremers (polysulfures d'aluminosilicates), le pyrophosphate de manganèse et certaines poudres métalliques telles que celles d'argent ou d'aluminium. On peut encore citer les laques couramment employées pour conférer aux lèvres et à la peau un effet de maquillage, qui sont des sels de calcium, de baryum, d'aluminium ou de zirconium, de colorants acides tels que les colorants halogéno-acides, azoïques, anthraquinoniques, etc.
Les nacres peuvent être présentes dans la composition à raison de 0 à 20% en poids, de préférence à un taux de l'ordre de 8 à 15% du poids total de la composition. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0 à 30%, de préférence 5 à 15% du poids total de la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le Polytrap (Dow Corning) et les microbilles de résine de silicone (Tospearls de Toshiba, par exemple).

La composition selon l'invention peut également comprendre au moins un composé éventuellement siliconé choisi parmi les cires de silicone, les résines de silicone, les huiles de silicone, éventuellement phénylées, un corps gras pâteux.

Ledit corps gras pâteux est de préférence hydrocarboné, peut être un polymère, et peut également être siliconé et/ou fluoré; il peut aussi se présenter sous forme d'un mélange de différents composés hydrocarbonés et/ou siliconés et/ou fluorés.

Dans le cas d'un mélange, on utilise de préférence les composés pâteux hydrocarbonés en proportion majoritaire.
On peut définir les composés gras pâteux selon l'invention à l'aide d'au moins une des propriétés physico-chimiques suivantes :

. une viscosité de 0,1 à 40 Pa.s (1 à 400 poises), de préférence 0,5 à 25 Pa.s, mesurée à 40°C avec un viscosimètre rotatif CONTRAVES TV équipé d'un mobile MS-r3 ou MS-r4 à la fréquence de 60 Hz,
. un point de fusion de 25-70°C, de préférence 25-55°C.

L'homme du métier peut choisir le mobile permettant de mesurer la viscosité, parmi les mobiles MS-r3 et MS-r4, sur base de ses connaissances générales, de manière à pouvoir réaliser la mesure du composé pâteux testé.
Parmi les composés pâteux susceptibles d'être utilisés dans le cadre de la présente invention, on peut citer les lanolines ou les dérivés de lanoline ayant une viscosité de 18-21 Pa.s, de préférence 19-20,5 Pa.s, et/ou un point de fusion de 30-60°C.
On peut également citer les esters gras, notamment ceux ayant 20 à 45 atomes de carbone (point de fusion de l'ordre de 25-70°C) ainsi que les triglycérides tels que les huiles végétales hydrogénées. Parmi les esters, on peut citer le propionate d'arachidyle, le polylaurate de vinyle et les esters de cholestérol.
On peut aussi citer les corps gras pâteux siliconés tels que les alkyls diméthicones, qui ont un point de fusion de 25-60°C, notamment ceux vendus par Dow Corning sous les noms commerciaux de DC2503 et DC25514.
On peut encore utiliser toute huile usuelle épaissie à l'aide d'un agent épaississant usuel.
Les huiles susceptibles d'être épaissies peuvent être d'origine minérale, végétale, animale et/ou synthétique telle que siliconée, et éventuellement phénylée.
L'agent épaississant peut être choisi parmi les argiles telles que les bentonites ou les hectorites, éventuellement modifiées notamment par du chlorure de distéaryl diméthyl ammonium, ou par du chlorure de stéaryl diméthyl benzyl ammonium, ou par des silicates d'aluminium ou de magnésium, ou encore par des polymères usuels connus pour être susceptibles d'épaissir des huiles.
On peut également utiliser les dérivés d'huile de ricin hydrogénée, tels que le 'THIXINR' de Rheox.
Le ou les composés pâteux peuvent être présents à raison de 1 à 40% en poids, de préférence à raison de 8-35% en poids et encore plus préférentiellement à raison de 15-30% en poids, par rapport au poids total de la composition.

Les huiles siliconées phénylées peuvent être un polyphénylméthylsiloxane ou un phényltriméthicone, ou un mélange de différentes huiles siliconées phénylées, et en particulier peuvent répondre à la formule suivante :

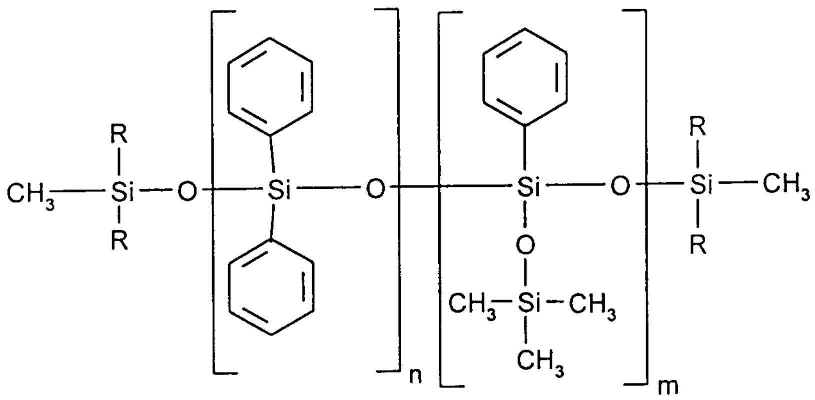

dans laquelle

. R est un radical alkyle en $C_1$-$C_{30}$, un radical aryle ou un radical aralkyle,
. n est un nombre entier compris entre 0 et 100,
. m est un nombre entier compris entre 0 et 100, sous réserve que la somme m+n est comprise entre 1 et 100.

De préférence, R est un radical méthyle, éthyle, propyle, isopropyle, décyle, dodécyle ou octadécyle, ou encore un radical phényle, tolyle, benzyle ou phénéthyle.

Parmi ces huiles phénylées, on peut citer l'huile Belsil PDM1000 de Wacker, les huiles DC556 ou SF558 de Dow Corning, l'huile Abil AV8853 de Goldschmidt ou l'huile Silbione 70633V30 de Rhône Poulenc.

Les cires de silicone doivent être solides ou semi-solides à température ambiante. Ces cires peuvent se présenter sous forme d'une pâte ou d'un solide rigide. En particulier, ces cires présentent une température de fusion supérieure à 25 °C et mieux supérieure à 45 °C.
Les cires de silicone de la composition de l'invention peuvent présenter la formule suivante :

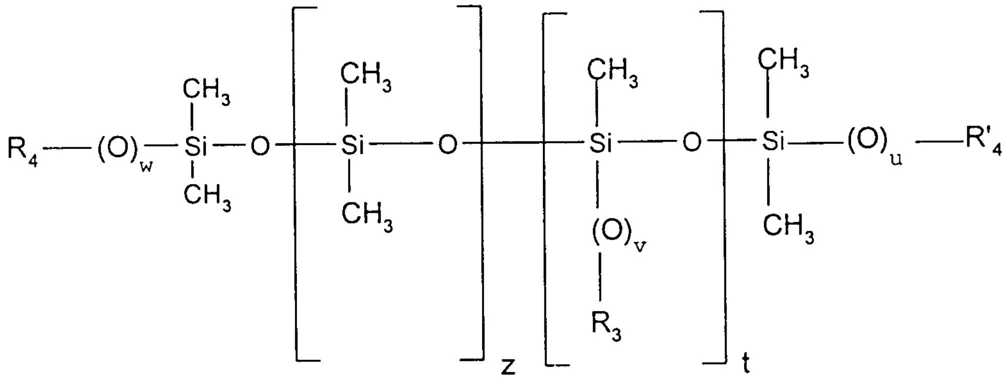

dans laquelle

- . $R_3$, $R_4$ et $R'_4$ représentent indépendamment l'un de l'autre un groupe méthyle ou hydrogène ou une chaîne alkyle linéaire ou ramifiée ayant de 10 à 45 atomes de carbone,
- . z et t représentent indépendamment un nombre entier allant de 0 à 100,
- . u, v et w représentent indépendamment 0 ou 1, à condition que t soit différent de 0 et $R_3$ différent de méthyle et hydrogène quand $R_4$ et $R'_4$ représentent le groupe méthyle ou hydrogène et que $R_4$ ou $R'_4$ soit différent du groupe méthyle ou hydrogène quand $R_3$ représente un groupe méthyle ou hydrogène ou quand t vaut 0.

En particulier, $R_3$, $R_4$ ou $R'_4$ représente un chaîne linéaire ayant 12 à 35 atomes de carbone, et mieux de 18 à 28 atomes de carbone comme par exemple les radicaux $C_{16}H_{33}$, $C_{18}H_{37}$, $C_{24}H_{49}$, $C_{26}H_{53}$, ou un mélange de ces radicaux. De préférence $R_3$ est une chaîne alkylée et $R_4$ le groupe méthyle, u, v, et w sont égaux à 0, z vaut de 2 à 40 et t vaut de 45 à 98.
Parmi les cires de silicone utilisables dans l'invention, on peut citer la béhenoxy dimethicone (avec $R_4$=$CH_3(CH_2)_{21}$, t=0, u=1, w=1, z<10) telle que celle vendue par Goldschmidt sous le nom Abil Wax 2440; la stearyl dimethicone (avec u=0, v=w=0 , $R_4$= $CH_3$ et $R_3$= stéaryle) telle que celle vendue par Dow Corning sous le nom DC 2503; la cétyl dimethicone (avec u=v=w=0 , $R_4$= $CH_3$ et $R_3$=cetyle) telle que celle vendue par Goldschmidt sous le nom Abil Wax 9814; la stearyl methicone (avec z=u=w=v=0 , $R_4$= CH3 et $R_3$=stearyle) telle que celle vendue par Goldschmidt sous le nom Abil Wax 9809; $C_{24}$-$C_{28}$ alkyl dimethicone (avec u=v=w=0 , $R_4$= $CH_3$ et $R_3$ est un groupe alkyle en $C_{24}$-$C_{28}$ et z<5) telle que celle vendue par Goldschmidt sous le nom Abil Wax 9810; $C_{30}$-$C_{45}$ alkyl methicone (avec z=u=v=w=0 , $R_4$= $CH_3$ et $R_3$=un groupe alkyle en $C_{30}$-$C_{45}$) telle que celle vendue par Goldschmidt sous le nom Abil Wax 9811; la stearoxy dimethicone (avec z=u=v=w=0 , $R_4$= $CH_3$ et $R_3$=stearyle) telle que celle vendue par Goldschmidt sous le nom Abil Wax 2434.
Comme autres cires de silicone utilisables dans l'invention, on peut citer les copolymères d'alkyl diméthicones.
Ces copolymères sont notamment ceux décrits dans les documents EP-A-527594, US-A-5 061 481, US-A- 5397 566, EP-A-527594 et peuvent présenter la formule suivante :

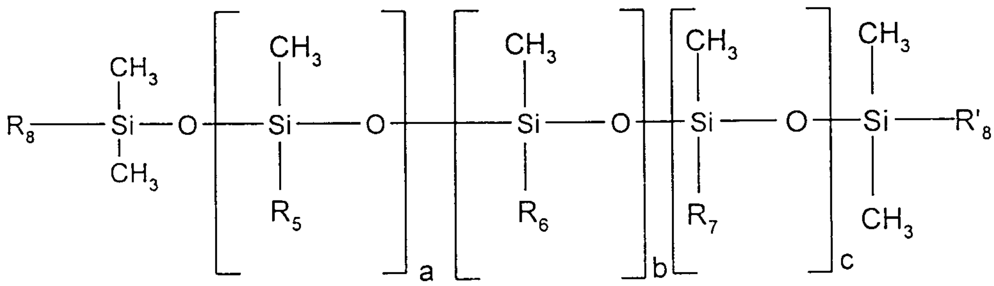

dans laquelle

- . $R_5$ ,$R_6$ ,$R_7$, $R_8$ et $R'_8$ représentent indépendamment l'un de l'autre un groupe méthyle ou hydrogène ou une chaîne alkyle ou alcoxy linéaire ou ramifiée ayant de 5 à 36 atomes de carbone,
- . a et b représentent indépendamment un nombre entier allant de 1 à 50 et
- . c représente un nombre entier allant de 0 à 50, à condition que deux des radicaux $R_5$, $R_6$, $R_7$, $R_8$ et $R'_8$ soient différents du groupe méthyle ou hydrogène et soient différents l'un de l'autre.

En particulier, $R_5$, $R_6$ représentent une chaîne linéaire ayant 10 à 20 atomes de carbone, avec $R_5$ différent de $R_6$, $R_8$ et $R'_8$ sont le groupe méthyle, a va de 8 à 18, b va de 2 à 12, c vaut 0.

L'efficacité remarquable des compositions démaquillante biphasiques est particulièrement évidente lorsque la composition est appliquée au démaquillage de compositions de maquillage sans transfert comprenant moins de 20% et plus particulièrement moins de 5% d'huiles non volatiles hydrocarbonées, et encore plus particulièrement à des compositions de maquillage ne comprenant pas d'huiles non volatiles hydrocarbonées, ces compositions étant connues pour leur excellente résistance au transfert.

Plus particulièrement, l'invention a pour objet l'utilisation d'une composition constituée d'une phase aqueuse et d'une phase huileuse distincte pour le démaquillage des compositions de rouge à lèvres sans transfert.

La composition démaquillante utilisée selon l'invention comprend au moins une phase aqueuse et une phase huileuse distincte.

La phase aqueuse de la composition démaquillante utilisée selon l'invention peut comprendre de l'eau déminéralisée stérile et/ou une eau florale telle que de l'eau de rose, de l'eau de bleuet, de l'eau de camomille ou de l'eau de tilleul, ou une eau thermale ou minérale naturelle, comme par exemple : l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avene.

La phase huileuse de la composition démaquillante selon l'invention, peut comprendre ou être constituée d'un mélange d'huiles, celles-ci pouvant être des huiles minérales, végétales ou synthétiques ou encore des huiles de silicone.

Parmi les huiles minérales pouvant constituer la phase huileuse, on peut notamment citer l'huile de vaseline et les hydrocarbures aliphatiques supérieurs tels que par exemple l'isohexadécane ; parmi les huiles végétales, l'huile de jojoba, ainsi que l'huile de carthame ; parmi les huiles de silicone éventuellement volatiles, le cyclopentadiméthylsiloxane vendu sous la dénomination de "VOLATIL SILICONE 7158" par la Société UNION CARBIDE et parmi les produits de synthèse, les palmitates d'alkyle ayant de 2 à 10 atomes de carbone tels que le palmitate d'isopropyle, ou le palmitate d'éthyl-2-hexyle et les adipates d'alkyle ayant de 2 à 10 atomes de carbone tels que l'adipate de di-éthyl-2-hexyle.

Selon une forme de réalisation particulière de l'invention, la phase huileuse contient au moins un palmitate d'alkyle ayant de 2 à 10 atomes de carbone en une proportion d'au moins 8 % et de préférence allant de 10 à 30 % par rapport au poids total de la composition démaquillante.

Selon une forme préférée de l'invention, la phase huileuse contient au moins une huile de silicone en une proportion d'au moins 8 % et de préférence allant de 15 à 50 % par rapport au poids total de la composition démaquillante.

De préférence, la composition biphase comprend en outre au moins un agent tensioactif dans l'une ou l'autre des phases.

L'agent tensioactif qui peut être du type anionique, non-ionique, ou amphotère mais de préférence du type non-ionique, est de préférence présent dans la phase aqueuse en une proportion allant de 0,1 à 10 % (de matière active) en poids

par rapport au poids total de la composition démaquillante, et encore plus préférentiellement de 0,5 à 3 %.

Parmi les agents tensioactifs non-ioniques, ceux particulièrement préférés sont :

- les esters gras de sorbitol polyoxyéthylénés tels que le produit vendu sous la dénomination de "TWEEN 20" par la Société ATLAS.
- les alcools gras polyoxyéthylénés tels que le produit vendu sous la dénomination de "REMCOPAL 21912 AL" par la Société GERLAND.
- les alkylphénols polyoxyéthylénés tels que le produit vendu sous la dénomination de "TRITON X 100" par la Société ROHM-HAAS, et
- les condensats d'oxyde d'éthylène et d'oxyde de propylène tels que ceux vendus sous les dénominations de "SYNPERONIC PE" par la Société ICI et en particulier ceux référencés L 31, L 64, F 38, F 88, L 92, P 103, F 108 et F 127.

Parmi les agents tensioactifs anioniques, on peut notamment citer :

- les alkyléthers sulfates tels que le produit vendu sous la dénomination de "TEXAPON ASV" par la Société HENKEL,
- les alkylsulfoacétates tels que produits vendu sous la dénomination de "LATHANOL LAL" par la Société STEPAN,
- les sulfosuccinates d'alkyle tels que le produit vendu sous la dénomination de "SODIUM DIOCTYL SULFOSUCCINATE" par la Société RHONE POULENC,
- les alkylamido sulfosuccinates tels que le produit vendu sous la dénomination de "REWODERM S 1333" par la Société REWO,
- les alkylamido polypeptides tels que le produit vendu sous la dénomination de "LAMEPON S" par la Société GRUNAU, et
- les acylsarcosinates tels que le produit vendu sous la dénomination de "ORAMIX L 30" par la Société SEPPIC.

Parmi les tensioactifs amphotères, on peut notamment citer :

- les alkylamidopropyl diméthylbétaïnes tels que le produit vendu sous la dénomination de "TEGO BETAINE L 7" par la Société GOLDSCHMIDT,
- les alkylamidobétaïnes tels que le produit vendu sous la dénomination de "INCRONAM 30" par la Société CRODA,
- les dérivés d'imidazoline tels que le produit vendu sous la dénomination de "CHIMEXANE HD" par la Société CHIMEX, et
- les N-alkyl-β-imino-dipropionates tels que le produit vendu sous la dénomination de "MONATERIC ISA 35" par la Société MONA.

De préférence, le rapport pondéral entre la phase aqueuse et la phase huileuse est compris entre 30/70 et 60/40.

La composition démaquillante utilisée selon l'invention peut également contenir des adjuvants cosmétiques conventionnels qui se trouveront dans l'une ou l'autre phase selon leur nature hydrophile ou lipophile, tels que par exemple des parfums, des agents conservateurs, dès colorants, des agents adoucissants, un tampon, des humectants et éventuellement un électrolyte tel que le chlorure de sodium pour apporter une isotonicité dans la phase aqueuse.

Parmi les agents humectants, on peut notamment mentionner, l'hexylèneglycol et le polyéthylèneglycol 600, ceux-ci étant présents à une concentration inférieure ou égale à 5% et de préférence allant de 0,05 à 2 % du poids total de la composition.

Parmi les agents adoucissants, on peut en particulier citer l'allantoïne, et certains extraits de plantes.

De telles compositions démaquillantes ont été décrites notamment dans le document EP-370856.

Selon l'invention, la composition démaquillante peut comprendre en outre de préférence dans la phase aqueuse un agent de déphasage en une proportion notamment allant de 0,025 à 5 % du poids total de la composition, ledit agent étant un chlorure d'alkyldiméthylbenzylammonium de formule :

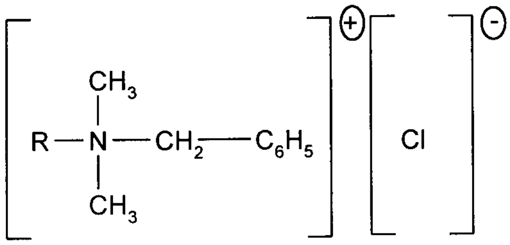

(I)

dans laquelle :

R représente un radical alkyle linéaire saturé ayant de 12 à 16 atomes de carbone, ou un mélange de chlorures d'alkyldiméthylbenzylammonium de formule (I), et/ou au moins 0,25% en poids par rapport au poids total de la composition d'un agent tensioactif, celui-ci étant du type anionique, non ionique ou amphotère lorsqu'il est présent dans la phase aqueuse ou du type non ionique liposoluble lorsqu'il est présent dans la phase huileuse.

De préférence, l'agent de déphasage utilisé dans la composition selon l'invention est un mélange de chlorure d'alkyldiméthylbenzylammonium de formule (I) constitué d'environ 65 % en poids de chlorure de lauryldiméthylbenzyl ammonium, d'environ 23 % en poids de chlorure de myristyldiméthylbenzylammonium, et d'environ 8 % en poids de chlorure de palmityldiméthylbenzylammonium, le reste étant constitué par au moins un chlorure d'alkyldiméthylammonium dont le radical a moins de 12 ou plus de 16 atomes de carbone.

Comme mélange de chlorures d'alkyldiméthylbenzylammonium utilisable selon l'invention, on peut citer celui commercialisé sous la dénomination de "chlorure de benzalkonium" par la Société FLUKA, dont les caractéristiques sont les suivantes : poids moléculaire = 360 et point de fusion = 35°C.

Le rapport entre l'agent tensioactif et l'agent de déphasage est de préférence compris entre 0,1/1 et 200/1. De telles compositions ont été décrites dans le document EP-603080.

Selon une variante de l'invention, on peut également prévoir que les compositions utilisées selon l'invention comprennent (a) une phase aqueuse comprenant un ou plusieurs agents tensioactifs, (b) une phase huileuse constituée de 50 à 100% d'un ou plusieurs dialkylphtalate(s). De préférence, on prévoit que ces compositions sont exemptes d'alcool.

La phase aqueuse est telle que décrite dans la première variante de l'invention.

Les dialkylphtalates utilisés dans les compositions ont pour formule générale :

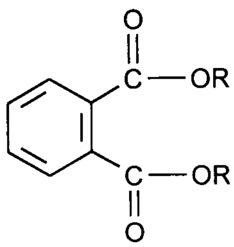

où R est un reste alkyle en $C_1$-$C_4$, en particulier un reste méthyle, éthyle, butyle.

Lorsque la teneur en dialkylphtalate de la phase huileuse est inférieure à 100% en poids, le complément à 100% est constitué par un ou plusieurs produits miscibles au dialkyphtalate.

Comme produits miscibles au dialkylphtalate, on peut citer les huiles, notamment les adipates tels que le dioctyladipate, les myristates tels que l'isopropylmyrisatate, les palmitates tels que l'octylpalmitate, les stéarates tels que l'isopropylstéarate, des vitamines telles que la vitamine A, la vitamine E, la vitamine F, des huiles telles que l'huile de tournesol, l'huile de poisson, le tétra éthyl-2 hexanoate de pentaérythritol et produits similaires.

En utilisant certains agents tensioactifs, on obtient des compositions cosmétiques esthétiquement attrayantes se présentant sous forme biphasée.

Lorsque l'agent tensioactif dissous dans la phase aqueuse est choisi parmi les agents tensioactifs anioniques, amphotères et zwittérioniques, la phase huileuse contenant le dialkylphtalate se trouve dispersée à l'agitation sous forme de microbilles dans la phase aqueuse, formant une suspension qui au repos se dépose au fond du récipient, produisant un effet poudreux.

Lorsque l'agent tensioactif en solution dans la phase aqueuse est choisi parmi les agents tensioactifs non-ioniques, la phase huileuse contenant le dialkylphtalate reste en suspension sous forme de microbilles dans la phase aqueuse, conférant ainsi à la composition un aspect laiteux.
La composition cosmétique utilisée selon cette variante de l'invention renferme de 0,5 à 20 % et de préférence de 2 à 10 % en poids de dialkylphtalate par rapport au poids total de la composition.
La quantité de l'agent tensioactif va de 0,1 à 30% en poids et de préférence de 2 à 6% en poids du poids total de la composition.
De telles compositions ont été décrites en particulier dans le brevet EP-490749.

Pour préparer la composition à deux phases, on peut préparer d'abord la phase aqueuse en dissolvant dans l'eau les adjuvants solubles dans l'eau. On peut disperser dans cette phase aqueuse, soit à froid, soit en chauffant légèrement, les particules solides insolubles dans l'eau et y verser ensuite la phase contenant le dialkylphtalate, les produits miscibles avec le dialkylphtalate et les autres adjuvants liposolubles (huiles, parfums, etc.). On peut agiter environ une heure à une heure et demi et en laissant reposer, on obtient deux phases lorsque l'on utilise des agents tensioactifs anioniques, amphotères ou zwittérioniques et une dispersion des deux phases lorsque l'on utilise un agent tensioactif non-ionique.

Selon une autre variante de l'invention, on peut prévoir que les compositions démaquillantes comprennent (a) une phase aqueuse, (b) une phase huileuse comprenant un ou plusieurs dialkylphtalate(s), (c) des particules solides insolubles dans la phase aqueuse et dans la phase huileuse et (d) un ou plusieurs agent(s) équilibrant les densités la phase huileuse se présentant sous la forme de billes qui, par agitation, se dispersent de façon homogène dans la phase aqueuse et au repos se reconstituent au fond du récipient sous forme de perles. Cette composition à deux phases, préférentiellement exempte d'alcool, présente un aspect esthétiquement attrayant. Des telles compositions ont été décrites dans le document EP-490 750.

Les particules solides insolubles dans la phase huileuse ainsi que dans la phase aqueuse, ont une dimension inférieure à 10 µm. On peut utiliser toute particule solide insoluble dans les deux phases et qui se maintient à l'interface huile/eau.
Les particules solides insolubles sont choisies de préférence dans le groupe formé par les matières minérales et organiques suivantes : oxyde de fer, dioxyde de titane, oxyde d'antimoine, oxyde de magnésium, alumine, oxyde de zinc, peroxyde de zinc, aluminate de calcium, acide silicique, silico-aluminate de magnésium, talc, mica, kaolin colloïdal, bentonite, laurate de zinc, chlorure de polyvinyle, nacre, noir de carbone, lanoline, et leurs mélanges.
Selon cette variante de l'invention, la composition cosmétique à deux phases comprend également un ou plusieurs agents équilibrant les densités qui ont pour but d'équilibrer les densités de la phase de dialkylphtalate et celle de la phase aqueuse. Ces agents équilibrant les densités sont choisis dans le groupe formé par les produits solubles dans la phase aqueuse, les produits solubles dans le dialkylphtalate et leurs mélanges. La fonction de l'agent équilibrant les densités est d'augmenter la densité de la phase aqueuse en augmentant sa masse sans pratiquement changer son volume ou bien de diminuer la densité du dialkylphtalate en augmentant son volume sans changer considérablement sa masse.
Comme exemple d'agent équilibrant les densités, soluble dans les dialkylphtalates, on peut citer les huiles, notamment les adipates tels que le dioctyladipate, les myristates tels que l'isopropylmyristate, les palmitates tels que l'octylpalmitate, les stéarates tels que l'isopropylstéarate, des vitamines telles que la vitamine A, la vitamine E, la vitamine F, des huiles telles que l'huile de tournesol, l'huile de poisson, le tétra éthyl-2 hexanoate de pentaérythritol et produits similaires.
Comme agent équilibrant les densités, soluble dans l'eau, on peut citer les sels minéraux ou organiques hydrosolubles tels que le phosphate trisodique, le phosphate disodique, le phosphate monosodique, le métabisulfite de sodium, le sulfate de magnésium, le sulfate de sodium, le phosphate monopotassique, le phosphate dipotassique, le phosphate tripotassique, le chlorure de sodium, le chlorure de potassium, les citrates mono-, di- et trisodiques, et en général les sels minéraux solubles dans l'eau.
Comme agent équilibrant les densités, soluble dans l'eau, on peut également utiliser un composant cosmétique, par exemple un conservateur, un filtre UV, un agent tampon, un agent d'éclat du teint et en général tout composé soluble dans l'eau qui augmente la densité de l'eau.
On peut utiliser à la fois un agent équilibrant les densités, soluble dans l'eau et un agent équilibrant les densités, soluble dans le dialkylphtalate.

Dans la composition liquide à deux phases, la phase à base de dialkylphtalate se présente sous forme de billes qui, par agitation, se dispersent sous forme de micro-billes dans la phase aqueuse et se reconstituent au repos au fond de la phase aqueuse. Les particules solides insolubles se placent à l'interface des billes d'huile contenant le dialkylphtalate et de l'eau.
Les particules solides insolubles et les agents équilibrant les densités favorisent la formation et la stabilité des billes d'huile dans l'eau au repos.

La composition cosmétique utilisée selon cette variante de l'invention renferme de 0,5 à 15 % et de préférence

de 2 à 10 % en poids de dialkylphtalate, de 1 à 10 % en poids d'agent équilibrant les densités et de 0,005 à 0,5 % et de préférence de 0,01 à 0,05 % en poids de particules solides insolubles dans les deux phases, du poids total de la composition. La phase aqueuse représente de 78 à 99,5 % et de préférence de 90 à 95 % en poids du poids total de la composition.

Pour préparer la composition à deux phases, on peut préparer d'abord la phase aqueuse en dissolvant dans l'eau l'agent équilibrant les densités et les adjuvants solubles dans l'eau. On peut disperser dans cette phase aqueuse, soit à froid, soit en chauffant légèrement, les particules solides insolubles dans l'eau et y verser ensuite la phase contenant le dialkylphtalate, les agents équilibrant la densité, solubles dans le dialkylphtalate et les autres adjuvants liposolubles (huiles, parfums, etc.). On peut agiter environ une heure à une heure et demi et en laissant reposer, on obtient deux phases.

Les compositions décrites ci-dessus peuvent être conditionnées, de façon connue, dans un flacon à un seul compartiment. L'utilisateur doit alors agiter le flacon avant d'en verser le contenu sur un coton. On peut également prévoir que les deux phases de la composition soient introduites dans deux compartiments indépendants d'un même flacon, un système étant prévu pour leur mélange au moment de la distribution. De tels dispositifs sont décrits par exemple dans les documents EP-A-497256 et FR-A-2697233.

On va maintenant donner à titre d'illustration, plusieurs exemples d'utilisation de compositions cosmétiques de démaquillage selon l'invention.

Des compositions démaquillantes biphasiques selon les différentes variantes décrites ci-dessus permettent d'obtenir un démaquillage parfait des compositions de rouge à lèvres et de fonds de teint sans transfert.

**Exemple 1: composition biphasique**

On prépare une composition comprenant :

| | |
|---|---|
| - cyclométhicone | 28 % |
| - isohexadécane | 19 % |
| - Poloxamer 184 (CTFA) | 0,05 % |
| - tampon phosphate | 0,15 % |
| - chlorure de sodium | 0,6 % |
| - conservateurs | qs |
| - colorants | qs |
| - eau déminéralisée | qsp 100 % |

**Exemple 2** (comparatif) : **lotion démaquillante monophasique**

On prépare une composition comprenant :

| | |
|---|---|
| - disodium cocoamphodiacétate | 0,4 % |
| - sodium laureth sulfate | 1 % |
| - sodium laureth-8 sulfate | 1 % |
| - allantoïne | 0,05 % |
| - hexylène glycol | 1 % |
| - chlorure de sodium | 1 % |
| - conservateurs | qs |
| - colorant | qs |
| - eau | qsp 100 % |

Les deux compositions (exemples 1 et 2) ont été testées par des utilisatrices (30 sujets testés) pour démaquiller deux compositions de rouge à lèvres sans transfert, commercialisées par la société L'OREAL sous le nom de ROUGE CAPTIVE, l'une de ces compositions étant de couleur violette et l'autre rouge. Chacune de ces utilisatrices devait indiquer pour chaque composition si elle présentait des propriétés de fraîcheur, de pouvoir démaquillant et de facilité de démaquillage.

Le résultat de ces essais est le suivant :

| | lotion monophasique exemple 2 | composition biphasique exemple 1 |
|---|---|---|
| fraîcheur | 79% | 46% |
| facilité de démaquillage | 80% | 93% |
| pouvoir démaquillant | 80% | 93% |

On constate par ces tests que la composition biphasique apporte moins de fraîcheur que la lotion. En revanche, elle se caractérise par une facilité de démaquillage et un pouvoir démaquillant supérieurs, de façon statistiquement significative, par rapport à la lotion.

<u>**Exemple 3** (comparatif) : **huile démaquillante**</u>

On prépare une composition comprenant :

| | |
|---|---|
| - cyclométhicone | 28 % |
| - isohexadécane | 19 % |
| - Poloxamer 184 | 0,05 % |

Cette huile démaquillante correspond à la phase huileuse des compositions selon l'invention.
On a comparé cette huile démaquillante avec la composition de l'exemple 1 pour leurs pouvoirs démaquillants et la facilité du démaquillage. Les utilisatrices portaient un rouge à lèvres sans transfert, commercialisé par la société L'OREAL sous le nom de ROUGE CAPTIVE et ont utilisé alternativement les compositions des exemples 1 et 3 pour se démaquiller.
L'huile démaquillante (exemple 3 comparatif) a été jugée trop lourde, tandis que, comparativement, la composition biphasique (Exemple 1) procure une agréable sensation de fraîcheur. L'huile démaquillante et la composition biphasique ont toutes deux un pouvoir démaquillant satisfaisant. Toutefois, l'huile démaquillante présente des difficultés d'utilisation : en effet, lors de son application à l'aide d'un coton, l'huile démaquillante 《 chasse 》 le rouge à lèvres et le fait déborder des lèvres, ce qui contraint l'utilisatrice à de nombreux passages de coton imprégné d'huile démaquillante.
Les utilisatrices ont jugé la facilité de démaquillage à l'aide de ces huiles non satisfaisante, tandis que la composition biphasique selon l'invention a été jugée très satisfaisante de ce point de vue.

## Revendications

1. Utilisation d'une composition constituée d'une phase aqueuse et d'une phase huileuse distinctes, pour le démaquillage des compositions de maquillage sans transfert.

2. Utilisation selon la revendication précédente, caractérisée en ce qu'elle s'applique au démaquillage d'un produit choisi parmi un fond de teint, un fard à joues ou à paupières et un rouge à lèvres.

3. Utilisation selon la revendication 1 ou 2, caractérisée en ce qu'elle s'applique au démaquillage d'un produit choisi parmi une composition de rouge à lèvres ou de fond de teint sans transfert.

**4.** Utilisation selon l'une quelconque des revendications précédentes, caractérisée en ce qu'elle s'applique au démaquillage de compositions de maquillage comprenant au moins une huile volatile, des corps gras et une phase particulaire.

**5.** Utilisation selon l'une quelconque des revendications précédentes, caractérisée en ce qu'elle s'applique au démaquillage de compositions de maquillage comprenant :

- de 1 à 90%, préférentiellement de 10 à 70% en poids d'une huile volatile,
- un composé éventuellement siliconé choisi parmi les cires de silicone, les résines de silicone, les huiles de silicone, éventuellement phénylées, un corps gras pâteux,
- jusqu'à 50% en poids d'une phase grasse constituée de corps gras usuels,
- de 0 à 30%, préférentiellement de 5 à 25% en poids d'une phase particulaire.

**6.** Utilisation selon l'une quelconque des revendications précédentes, caractérisée en ce qu'elle s'applique au démaquillage de compositions de maquillage comprenant moins de 20%, plus particulièrement moins de 5% d'huiles non volatiles hydrocarbonées.

**7.** Utilisation selon l'une quelconque des revendications précédentes, caractérisée en ce qu'elle s'applique au démaquillage de compositions de maquillage ne comprenant pas d'huiles non volatiles hydrocarbonées.

**8.** Utilisation selon l'une quelconque des revendications précédentes, caractérisée en ce qu'elle s'applique au démaquillage de compositions de rouge à lèvres sans transfert.

**9.** Utilisation selon l'une quelconque des revendications précédentes, caractérisée en ce que la phase aqueuse de la composition démaquillante comprend de l'eau déminéralisée stérile et/ou une eau florale telle que l'eau de rose, l'eau de bleuet, l'eau de camomille ou l'eau de tilleul, et/ou une eau thermale ou minérale naturelle.

**10.** Utilisation selon l'une quelconque des revendications précédentes, caractérisée en ce que la phase huileuse de la composition démaquillante comprend au moins une huile choisie parmi l'huile de vaseline, les hydrocarbures aliphatiques supérieurs tels que par exemple l'isohexadécane, les huiles végétales telles que l'huile de jojoba et l'huile de carthame, les huiles de silicone éventuellement volatiles, les palmitates d'alkyle ayant de 2 à 10 atomes de carbone, les adipates d'alkyle ayant de 2 à 10 atomes de carbone.

**11.** Utilisation selon l'une quelconque des revendications précédentes, caractérisée en ce que la phase huileuse de la composition démaquillante comprend au moins un palmitate d'alkyle ayant de 2 à 10 atomes de carbone en une proportion d'au moins 8 % en poids par rapport au poids total de la composition.

**12.** Utilisation selon l'une quelconque des revendications précédentes, caractérisée en ce que la phase huileuse de la composition démaquillante comprend au moins une huile de silicone en une proportion d'au moins 8 % en poids par rapport au poids total de la composition démaquillante.

**13.** Utilisation selon l'une quelconque des revendications précédentes, caractérisée en ce que la composition biphase comprend au moins un agent tensioactif, de préférence choisi parmi les tensioactifs anionique, non-ionique et amphotère.

**14.** Utilisation selon la revendication 13, caractérisée en ce que l'agent tensioactif est non-ionique.

**15.** Utilisation selon l'une quelconque des revendications 13 à 14, caractérisée en ce que l'agent tensioactif est présent dans la phase aqueuse en une proportion allant de 0,1 à 10 % en poids par rapport au poids total de la composition.

**16.** Utilisation selon l'une quelconque des revendications précédentes, caractérisée en ce que le rapport pondéral entre la phase aqueuse et la phase huileuse est compris entre 30/70 et 60/40.

**17.** Utilisation selon l'une quelconque des revendications précédentes, caractérisée en ce que la composition démaquillante comprend en outre au moins un agent de déphasage en une proportion allant de 0,025 à 5 % du poids total de la composition, ledit agent étant un chlorure d'alkyldiméthylbenzylammonium de formule :

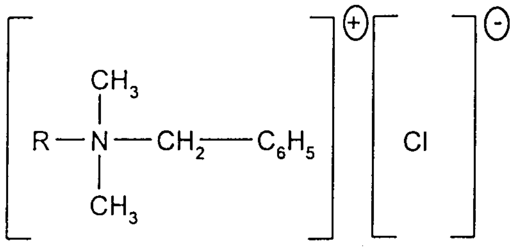 (I)

dans laquelle R représente un radical alkyle linéaire saturé ayant de 12 à 16 atomes de carbone, et/ ou au moins 0,25% en poids par rapport au poids total de la composition d'un agent tensioactif.

**18.** Utilisation selon la revendication 17, caractérisée en ce que l'agent de déphasage est un mélange de chlorure d'alkyldiméthylbenzylammonium de formule (I), constitué d'environ 65 % en poids de chlorure de lauryldiméthylbenzyl ammonium, d'environ 23 % en poids de chlorure de myristyldiméthyl-benzylammonium, et d'environ 8 % en poids de chlorure de palmityldiméthyl-benzylammonium, le reste étant constitué par au moins un chlorure d'alkyl-diméthylammonium dont le radical a moins de 12 ou plus de 16 atomes de carbone.

**19.** Utilisation selon l'une quelconque des revendications 17 à 18, caractérisée en ce que le rapport entre l'agent tensioactif et l'agent de déphasage est compris entre 0,1/1 et 200/1.

**20.** Utilisation selon l'une quelconque des revendications 1 à 9, caractérisée en ce que la composition démaquillante comprend (a) une phase aqueuse comprenant au moins un tensioactif, (b) une phase huileuse constituée de 50 à 100% en poids d'un ou plusieurs dialkylphtalate(s) de formule générale :

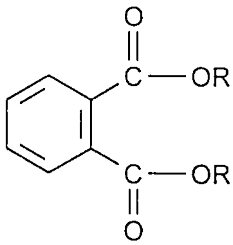

où R est un reste alkyle en $C_1$-$C_4$, en particulier un reste méthyle, éthyle, butyle.

**21.** Utilisation selon la revendication 20, caractérisée en ce que la teneur en dialkylphtalate de la phase huileuse est de 100%.

**22.** Utilisation selon l'une quelconque des revendications 20 à 21, caractérisée en ce que la composition démaquillante comprend de 0,5 à 20 % et de préférence de 2 à 10 % en poids de dialkylphtalate, par rapport au poids total de la composition.

**23.** Utilisation selon l'une quelconque des revendications 20 à 22, caractérisée en ce que la composition démaquillante comprend de 0,1 à 30 % et de préférence de 2 à 6% du poids total de la composition, d'au moins un agent tensioactif.

**24.** Utilisation selon l'une quelconque des revendications 1 à 9, caractérisée en ce que la composition démaquillante comprend (a) une phase aqueuse, (b) une phase huileuse comprenant un ou plusieurs dialkylphtalate(s) de formule générale :

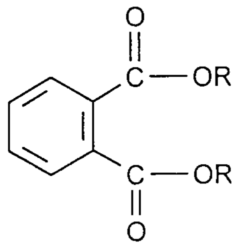

où R est un reste alkyle en $C_1$-$C_4$, en particulier un reste méthyle, éthyle, butyle, (c) des particules solides insolubles dans la phase aqueuse et dans la phase huileuse et (d) un ou plusieurs agent(s) équilibrant les densités.

**25.** Utilisation selon la revendication 24, caractérisée en ce que les particules solides insolubles sont choisies parmi les matières minérales et organiques suivantes : oxyde de fer, dioxyde de titane, oxyde d'antimoine, oxyde de magnésium, alumine, oxyde de zinc, peroxyde de zinc, aluminate de calcium, acide silicique, silico-aluminate de magnésium, talc, mica, kaolin colloïdal, bentonite, laurate de zinc, chlorure de polyvinyle, nacre, noir de carbone, lanoline et leurs mélanges.

**26.** Utilisation selon l'une quelconque des revendications 24 à 25, caractérisée en ce que l'agent équilibrant les densités est choisi dans le groupe formé par les adipates, les myristates, les palmitates, les stéarates, les vitamines, des huiles, le tétra-éthyl-2 hexanoate de pentaérythritol, le phosphate trisodique, le phosphate disodique, le phosphate monosodique, le métabisulfite de sodium, le sulfate de magnésium, le sulfate de sodium, le phosphate monopotassique, le phosphate dipotassique, le phosphate tripotassique, le chlorure de sodium, le chlorure de potassium, les citrates mono-, di- et trisodiques, et les sels minéraux solubles dans l'eau, un conservateur, un filtre UV, un agent tampon, un agent d'éclat du teint, leurs mélanges.

**27.** Utilisation selon l'une quelconque des revendications 24 à 26, caractérisée en ce que la composition démaquillante comprend de 0,5 à 15 % et de préférence de 2 à 10 % en poids de dialkylphtalate, par rapport au poids total de la composition.

**28.** Utilisation selon l'une quelconque des revendications 24 à 27, caractérisée en ce que la composition démaquillante comprend de 1 à 10 % en poids d'agent équilibrant les densités, par rapport au poids total de la composition.

**29.** Utilisation selon l'une quelconque des revendications 24 à 28, caractérisée en ce que la composition démaquillante comprend de 0,005 à 0,5 % et de préférence de 0,01 à 0,05 % en poids de particules solides insolubles dans les deux phases, par rapport au poids total de la composition.

## Claims

**1.** Use of a composition consisting of an aqueous phase and of an oily phase, which are distinct, for the removal of transfer-free make-up compositions.

**2.** Use according to the preceding claim, characterized in that it applies to the removal of make-up of a product chosen from a foundation, a blusher or eye shadow and a lip rouge.

**3.** Use according to Claim 1 or 2, characterized in that it applies to the removal of make-up of a product chosen from a lip rouge composition or transfer-free foundation.

**4.** Use according to any one of the preceding claims, characterized in that it applies to the removal of make-up of make-up compositions including at least one volatile oil, fatty substances and a particulate phase.

**5.** Use according to any one of the preceding claims, characterized in that it applies to the removal of make-up of make-up compositions including:

- from 1 to 90 %, preferably from 10 to 70 %, by weight of a volatile oil,
- an optionally silicone-containing compound chosen from silicone waxes, silicone resins, silicone oils, optionally

phenylated, and a pasty fatty substance,

- up to 50 % by weight of a fatty phase consisting of conventional fatty substances,
- from 0 to 30 %, preferably from 5 to 25 %, by weight of a particulate phase.

**6.** Use according to any one of the preceding claims, characterized in that it applies to the removal of make-up of make-up compositions including less than 20 %, more particularly less than 5 %, of nonvolatile hydrocarbon oils.

**7.** Use according to any one of the preceding claims, characterized in that it applies to the removal of make-up of make-up compositions not including any nonvolatile hydrocarbon oils.

**8.** Use according to any one of the preceding claims, characterized in that it applies to the removal of make-up of transfer-free lip rouge compositions.

**9.** Use according to any one of the preceding claims, characterized in that the aqueous phase of the make-up removal composition includes sterile demineralized water and/or a floral water such as rose water, cornflower water, camomile water or lime water and or a natural thermal or mineral water.

**10.** Use according to any one of the preceding claims, characterized in that the oily phase of the make-up removal composition includes at least one oil chosen from liquid petrolatum, higher aliphatic hydrocarbons such as, for example, isohexadecane, vegetable oils such as jojoba oil and safflower oil, optionally volatile silicone oils, alkyl palmitates, the alkyl group containing from 2 to 10 carbon atoms, and alkyl adipates, the alkyl group containing from 2 to 10 carbon atoms.

**11.** Use according to any one of the preceding claims, characterized in that the oily phase of the make-up removal composition includes at least one alkyl palmitate, the alkyl group containing from 2 to 10 carbon atoms, in a proportion of at least 8 % by weight relative to the total weight of the composition.

**12.** Use according to any one of the preceding claims, characterized in that the oily phase of the make-up removal composition includes at least one silicone oil in a proportion of at least 8 % by weight relative to the total weight of the make-up removal composition.

**13.** Use according to any one of the preceding claims, characterized in that the two-phase composition includes at least one surface-active agent preferably chosen from anionic, nonionic and amphoteric surfactants.

**14.** Use according to Claim 13, characterized in that the surface-active agent is nonionic.

**15.** Use according to either of Claims 13 and 14, characterized in that the surface-active agent is present in the aqueous phase in a proportion ranging from 0.1 to 10 % by weight relative to the total weight of the composition.

**16.** Use according to any one of the preceding claims, characterized in that the weight ratio of the aqueous phase and the oily phase is between 30/70 and 60/40.

**17.** Use according to any one of the preceding claims, characterized in that the make-up removal composition additionally includes at least one dephasing agent in a proportion ranging from 0.025 % to 5 % of the total weight of the composition, the said agent being an alkyldimethylbenzylammonium chloride of formula:

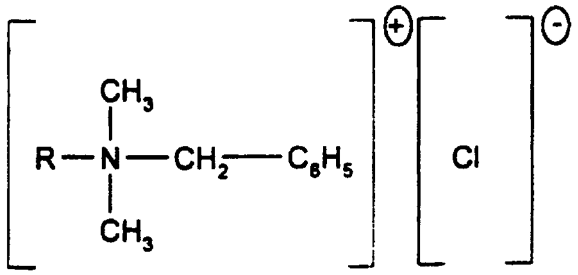

(I)

in which R denotes a saturated linear alkyl radical containing from 12 to 16 carbon atoms and/or at least 0.25 % by

weight, relative to the total weight of the composition, of a surface-active agent.

**18.** Use according to Claim 17, characterized in that the dephasing agent is a mixture of alkyldimethylbenzylammonium chloride of formula (I), consisting of approximately 65 % by weight of lauryldimethylbenzylammonium chloride, approximately 23 % by weight of myristyldimethylbenzylammonium chloride and approximately 8 % by weight of palmityldimethylbenzylammonium chloride, the remainder consisting of at least one alkyldimethylammonium chloride in which the radical has fewer than 12 or more than 16 carbon atoms.

**19.** Use according to either of Claims 17 and 18, characterized in that the ratio of the surface-active agent and of the dephasing agent is between 0.1/1 and 200/1.

**20.** Use according to any one of Claims 1 to 9, characterized in that the make-up removal composition includes (a) an aqueous phase including at least one surfactant, (b) an oily phase consisting of 50 to 100 % by weight of one or more dialkyl phthalate(s) of general formula:

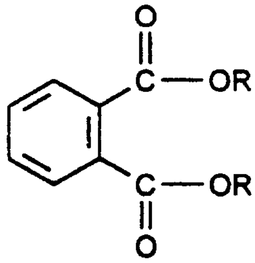

where R is a $C_1$-$C_4$ alkyl residue, in particular a methyl, ethyl or butyl residue.

**21.** Use according to Claim 20, characterized in that the dialkyl phthalate content of the oily phase is 100 %.

**22.** Use according to either of Claims 20 and 21, characterized in that the make-up removal composition includes from 0.5 to 20 % and preferably from 2 to 10 % by weight of dialkyl phthalate, relative to the total weight of the composition.

**23.** Use according to any one of Claims 20 to 22, characterized in that the make-up removal composition includes from 0.1 to 30 % and preferably from 2 to 6 % of the total weight of the composition of at least one surface-active agent.

**24.** Use according to any one of Claims 1 to 9, characterized in that the make-up removal composition includes (a) an aqueous phase, (b) an oily phase including one or more dialkyl phthalate(s) of general formula:

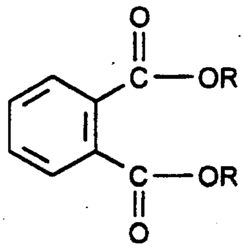

where R is a $C_1$-$C_4$ alkyl residue, in particular a methyl, ethyl or butyl residue, (c) solid particles which are insoluble in the aqueous phase and in the oily phase, and (d) one or more agent(s) equilibrating the densities.

**25.** Use according to Claim 24, characterized in that the insoluble solid particles are chosen from the following inorganic and organic materials: iron oxide, titanium dioxide, antimony oxide, magnesium oxide, alumina, zinc oxide, zinc peroxide, calcium aluminate, silicic acid, magnesium silicoaluminate, talc, mica, colloidal kaolin, bentonite, zinc laurate, polyvinyl chloride, mother-of-pearl, carbon black, lanolin and mixtures thereof.

**26.** Use according to either of Claims 24 and 25, characterized in that the agent equilibrating the densities is chosen

from the group made up of adipates, myristates, palmitates, stearates, vitamins, oils, pentaerythritol tetra-2-ethylhexanoate, trisodium phosphate, disodium phosphate, monosodium phosphate, sodium metabisulphite, magnesium sulphate, sodium sulphate, monopotassium phosphate, dipotassium phosphate, tripotassium phosphate, sodium chloride, potassium chloride, mono-, di- and trisodium citrates, and water-soluble inorganic salts, a preserving agent, a UV screening agent, a buffer, a complexion radiance agent and mixtures thereof.

**27.** Use according to any one of Claims 24 to 26, characterized in that the make-up removal composition includes from 0.5 to 15 % and preferably from 2 to 10 % by weight of dialkyl phthalate, relative to the total weight of the composition.

**28.** Use according to any one-of Claims 24 to 27, characterized in that the make-up removal composition includes from 1 to 10 % by weight of agent equilibrating the densities, relative to the total weight of the composition.

**29.** Use according to any one of Claims 24 to 28, characterized in that the make-up removal composition includes from 0.005 to 0.5 % and preferably from 0.01 to 0.05 % by weight of solid particles which are insoluble in both phases, relative to the total weight of the composition.

**Patentansprüche**

**1.** Verwendung einer Zusammensetzung, die aus einer wäßrigen Phase und einer davon verschiedenen Ölphase besteht, zum Abschminken von Schminkzusammensetzungen "ohne Transfer".

**2.** Verwendung nach dem vorhergehenden Anspruch, dadurch gekennzeichnet, daß sie Anwendung beim Abschminken eines Produkts findet, das unter Make-up, Rouge, Lidschatten und Lippenstift ausgewählt wird.

**3.** Verwendung nach Anspruch 1 oder 2, dadurch gekennzeichnet, daß sie Anwendung beim Abschminken einer Lippenstiftzusammensetzung oder einer Make-up-Zusammensetzung "ohne Transfer" findet.

**4.** Verwendung nach einem der vorhergehenden Ansprüche, dadurch gekennzeichnet, daß sie Anwendung beim Abschminken von Schminkzusammensetzungen findet, die mindestens ein flüchtiges Öl, Fettsubstanzen und eine Partikelphase enthalten.

**5.** Verwendung nach einem der vorhergehenden Ansprüche, dadurch gekennzeichnet, daß sie Anwendung beim Abschminken von Schminkzusammensetzungen findet, die enthalten:

- 1 bis 90 Gew.-%, vorzugsweise 10 bis 70 Gew.-%, eines flüchtigen Öls,
- eine gegebenenfalls siliconhaltige Verbindung, die ausgewählt ist unter Siliconwachsen, Siliconharzen, Siliconölen, die gegebenenfalls phenylgruppenhaltig sind, eine pastöse Fettsubstanz,
- bis zu 50 Gew.-% einer Fettphase, die aus herkömmlichen Fettsubstanzen besteht,
- 0 bis 30 Gew.-%, vorzugsweise 5 bis 25 Gew.-%, einer Partikelphase.

**6.** Verwendung nach einem der vorhergehenden Ansprüche, dadurch gekennzeichnet, daß sie Anwendung beim Abschminken von Schminkzusammensetzungen findet, die weniger als 20 % und insbesondere weniger als 5 % nicht flüchtige Kohlenwasserstofföle enthalten.

**7.** Verwendung nach einem der vorhergehenden Ansprüche, dadurch gekennzeichnet, daß sie Anwendung beim Abschminken von Schminkzusammensetzungen findet, die keine nicht flüchtigen Kohlenwasserstofföle enthalten.

**8.** Verwendung nach einem der vorhergehenden Ansprüche, dadurch gekennzeichnet, daß sie Anwendung beim Abschminken von Lippenstiftzusammensetzungen "ohne Transfer" findet.

**9.** Verwendung nach einem der vorhergehenden Ansprüche, dadurch gekennzeichnet, daß die wäßrige Phase der Abschminkzusammensetzung steriles entmineralisiertes Wasser und/oder ein Blütenwasser, wie z.B. Rosenwasser, Kornblumenwasser, Kamillenwasser oder Lindenblütenwasser, und/oder ein Thermalwasser und/oder ein natürliches Mineralwasser enthält.

**10.** Verwendung nach einem der vorhergehenden Ansprüche, dadurch gekennzeichnet, daß die Ölphase der Abschminkzusammensetzung mindestens ein Öl enthält, das unter Vaselineöl, höheren aliphatischen Kohlenwasserstoffen wie Isohexadecan, pflanzlichen Ölen wie Jojobaöl sowie Safloröl, gegebenenfalls flüchtigen Siliconölen, Alkylpalmitaten mit 2 bis 10 Kohlenstoffatomen, Alkyladipaten mit 2 bis 10 Kohlenstoffatomen ausgewählt wird.

**11.** Verwendung nach einem der vorhergehenden Ansprüche, dadurch gekennzeichnet, daß die Ölphase der Abschminkzusammensetzung mindestens ein Alkylpalmitat mit 2 bis 10 Kohlenstoffatomen in einem Anteil von mindestens 8 %, bezogen auf das Gesamtgewicht der Abschminkzusammensetzung, enthält.

**12.** Verwendung nach einem der vorhergehenden Ansprüche, dadurch gekennzeichnet, daß die Ölphase der Abschminkzusammensetzung mindestens ein Siliconöl in einem Anteil von mindestens 8 %, bezogen auf das Gesamtgewicht der Abschminkzusammensetzung, enthält.

**13.** Verwendung nach einem der vorhergehenden Ansprüche, dadurch gekennzeichnet, daß die zweiphasige Zusammensetzung mindestens einen grenzflächenaktiven Stoff enthält, der vorzugsweise unter anionischen, nichtionischen und amphoteren grenzflächenaktiven Stoffen ausgewählt wird.

**14.** Verwendung nach Anspruch 13, dadurch gekennzeichnet, daß der grenzflächenaktive Stoff nichtionisch ist.

**15.** Verwendung nach einem der Ansprüche 13 und 14, dadurch gekennzeichnet, daß der grenzflächenaktive Stoff in der wäßrigen Phase in einem Anteil von 0,1 bis 10 Gew.-%, bezogen auf das Gesamtgewicht der Zusammensetzung, enthalten ist.

**16.** Verwendung nach einem der vorhergehenden Ansprüche, dadurch gekennzeichnet, daß das Gewichtsverhältnis wäßrige Phase zu Ölphase im Bereich von 30/70 bis 60/40 liegt.

**17.** Verwendung nach einem der vorhergehenden Ansprüche, dadurch gekennzeichnet, daß die Abschminkzusammensetzung außerdem mindestens ein Phasentrennmittel in einem Anteil von 0,025 bis 5 % des Gesamtgewichts der Zusammensetzung, wobei es sich bei dem Mittel um ein Alkyldimethylbenzylammoniumchlorid der Formel

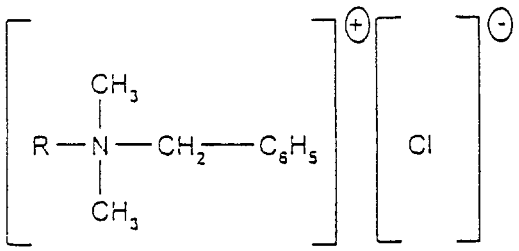

(I)

handelt, in der R gesättigtes geradkettiges Alkyl mit 12 bis 16 Kohlenstoffatomen bedeutet,
und/oder mindestens 0,25 Gew.-%, bezogen auf das Gesamtgewicht der Zusammensetzung, eines grenzflächenaktiven Stoffs enthält.

**18.** Verwendung nach Anspruch 17, dadurch gekennzeichnet, daß das Phasentrennmittel ein Gemisch von Alkyldimethylbenzylammoniumchloriden der Formel (I) ist, das aus etwa 65 Gew.-% Lauryldimethylbenzylammoniumchlorid, etwa 23 Gew.-% Myristyldimethylbenzylammoniumchlorid und etwa 8 Gew.-% Palmityldimethylbenzylammoniumchlorid besteht, wobei der Rest aus mindestens einem Alkyldimethylammoniumchlorid besteht, dessen Alkylgruppe mindestens 12 oder höchstens 16 Kohlenstoffatome aufweist.

**19.** Verwendung nach einem der Ansprüche 17 und 18, dadurch gekennzeichnet, daß das Verhältnis grenzflächenaktiver Stoff zu Phasentrennmittel im Bereich von 0,1/1 bis 200/1 liegt.

**20.** Verwendung nach einem der Ansprüche 1 bis 9, dadurch gekennzeichnet, daß die Abschminkzusammensetzung enthält: (a) eine wäßrige Phase, die mindestens einen grenzflächenaktive Stoffe enthält, (b) eine Ölphase, die zu 50 bis 100 % aus einem oder mehreren Dialkylphthalaten der allgemeine Formel

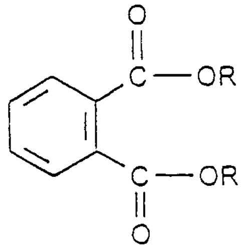

besteht, in der R $C_1$-$C_4$-Alkyl und insbesondere Methyl, Ethyl, Butyl bedeutet.

**21.** Verwendung nach Anspruch 20, dadurch gekennzeichnet, daß der Gehalt der Ölphase an Dialkylphthalat 100 % beträgt.

**22.** Verwendung nach Anspruch 20 oder 21, dadurch gekennzeichnet, daß die Abschminkzusammensetzung 0,5 bis 20 Gew.-% und vorzugsweise 2 bis 10 Gew.-% Dialkylphthalat, bezogen auf das Gesamtgewicht der Zusammensetzung, enthält.

**23.** Verwendung nach einem der Ansprüche 20 bis 22, dadurch gekennzeichnet, daß die Abschminkzusammensetzung 0,1 bis 30 Gew.-% und vorzugsweise 2 bis 6 Gew.-% des Gesamtgewichts der Zusammensetzung mindestens eines grenzflächenaktiven Stoffs enthält.

**24.** Verwendung nach einem der Ansprüche 1 bis 9, dadurch gekennzeichnet, daß die Abschminkzusammensetzung enthält: (a) eine wäßrige Phase, (b) eine Ölphase, die ein oder mehrere Dialkylphthalate der allgemeinen Formel

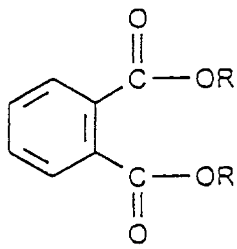

enthält, in der R $C_1$-$C_4$-Alkyl und insbesondere Methyl, Ethyl, Butyl bedeutet, (c) feste Partikel, die in der wäßrigen Phase und der Ölphase unlöslich sind, und (d) ein oder mehrere die Dichten angleichende Mittel.

**25.** Verwendung nach Anspruch 24, dadurch gekennzeichnet, daß die unlöslichen festen Partikel unter den folgenden anorganischen und organischen Materialien ausgewählt werden: Eisenoxid, Titandioxid, Antimonoxid, Magnesiumoxid, Aluminiumoxid, Zinkoxid, Zinkperoxid, Calciumaluminat, Kieselsäure, Magnesiumaluminiumsilicat, Talk, Glimmer, kolloidalem Kaolin, Bentonit, Zinklaurat, Polyvinylchlorid, Perlmutt, Perlglanzpigmenten, Ruß, Lanolin und Gemische dieser Stoffe.

**26.** Verwendung nach Anspruch 24 oder 25, dadurch gekennzeichnet, daß das die Dichten angleichende Mittel ausgewählt wird unter: Adipaten, Myristaten, Palmitaten, Stearaten, Vitaminen, Ölen, Pentaerythrit-tetra-2-ethylhexanoat, Trinatriumphosphat, Dinatriumphosphat, Mononatriumphosphat, Natriummetabisulfit, Magnesiumsulfat, Natriumsulfat, Monokaliumphosphat, Dikaliumphosphat, Trikaliumphosphat, Natriumchlorid, Kaliumchlorid, Mono-, Di- und Trinatriumcitrat, in Wasser löslichen anorganischen Salzen, Konservierungsmitteln, UV-Filtern, Puffern, Mitteln für einen helleren oder glänzenden Teint und ihren Gemischen.

**27.** Verwendung nach einem der Ansprüche 24 bis 26, dadurch gekennzeichnet, daß die Abschminkzusammensetzung 0,5 bis 15 Gew.-% und vorzugsweise 2 bis 10 Gew.-% Dialkylphthalat, bezogen auf das Gesamtgewicht der Zusammensetzung, enthält.

**28.** Verwendung nach einem der Ansprüche 24 bis 27, dadurch gekennzeichnet, daß die Abschminkzusammensetzung 1 bis 10 Gew.-% die Dichten angleichendes Mittel, bezogen auf das Gesamtgewicht der Zusammensetzung, enthält.

**29.** Verwendung nach einem der Ansprüche 24 bis 28, dadurch gekennzeichnet, daß die Abschminkzusammensetzung 0,005 bis 0,5 Gew.-% und vorzugswseise 0,01 bis 0,05 Gew.-% in den beiden Phasen unlösliche feste Partikel, bezogen auf das Gesamtgewicht der Zusammensetzung, enthält.